# EUROPEAN PATENT SPECIFICATION

(11) **EP 1 684 677 B1**
(45) Date of publication and mention of the grant of the patent: **05.05.2010**
(21) Application number: 04791931.1
(22) Date of filing: 29.10.2004
(51) Int. Cl.: A61F 5/01, A61H 1/02

(54) **ARTICULATED JOINT FOR KNEE BRACE COMPRISING MEANS FOR OPERATING THE SAME**
SCHARNIERGELENK FÜR EINE KNIESCHIENE MIT EINEM MITTEL ZU SEINER BEDIENUNG
ARTICULATION DESTINEE A UNE ORTHESE POUR LE GENOU QUI COMPREND UN SYSTEME D'OPERATION CORRESPONDANT

(30) Priority: 04.11.2003 IT VR20030129
(43) Date of publication of application: 02.08.2006
(73) Proprietor: F.G.P. Srl, 37062 Dossobuono (VR) (IT)
(72) Inventor: TURRINI, Alberto, I-37060 Castel d'Azzano (IT); FERRIGOLO, Moreno, I-37062 Dossobuono (IT)
(74) Representative: Petraz, Gilberto Luigi
(86) International application number: PCT/IT2004/000597
(87) International publication number: WO 2005/046536

(56) References cited:
- EP-A- 0 475 735
- EP-A- 0 510 840
- WO-A-02/092164
- US-A- 3 449 769
- US-A- 4 538 595
- US-A- 5 683 351

## Description

### TECHNICAL FIELD

This invention concerns an articulated joint for a knee brace or for articular walkers in general, with the particular feature of being equipped with power-driven operating means.

More in particular, this invention refers to an articulated joint for a knee brace which is designed to be fitted with a power-driven system that makes it possible to mechanically and automatically vary the opening angle of the two uprights which it joins together.

This power-driven system uses an electrical type of motor, which can be connected to a control unit or to a PLC, and can be fitted on the brace in an operating position kinematically connected to one of the uprights.

The use of the power-driven system fitted to the orthopaedic walker allows the walker to be used for rehabilitation exercises of the limb after surgery or as part of articular excursion rehabilitation therapy.

This invention can be applied in the industry for the production of orthopaedic equipment and in particular equipment such as knee braces or the like to control isometric flexure of the limbs.

### BACKGROUND ART

It is known that following surgery to the arms and legs it is necessary for the patient to wear auxiliary equipment or ortheses, that is to say fixed or mobile equipment that increases, improves or controls the impaired function of body parts, such as walkers or the like, and to undergo rehabilitation cycles to restore the limb to perfect condition.

After an operation on the ligaments or cartilaginous membranes, the limb is kept in a blocked position, first totally then partially, by means of special orthopaedic equipment generally called walkers or knee braces, which assist and support the weakened joint, absorbing the most intense stress.

The first exercises recommended are passive flexure-extension and circumduction movements: these movements are very useful in stimulating the circulation of the limb and activating the muscular structure.

In the last few years, closed kinematic chain exercises, in which both the proximal and distal ends of the system are blocked, have been widely employed to optimise the initial healing phase of ligament transplants.

In order to perform flexure-extension limb rehabilitation exercises, the devices currently used allow passive movement of the joint. These devices allow the limb to be stretched out in a substantially horizontal position, while the person is sitting or lying down, and to be flexed with a progressive increase of the angle as the rehabilitation programme proceeds.

These kinetic type devices normally consist of a frame with support means for the leg or the arm, which are kept in an extended position, and adjustable angle thrust or flexure means, with excursions from 0 to 90 and 120°.

These devices are normally used while the person is lying down or sitting, and therefore, in addition to being fairly complex and cumbersome, cannot be used in other positions.

### DESCRIPTION OF THE INVENTION

Document WO-A2-02/092164 discloses a therapeutic and/or training device for a person's lower limbs comprising a mechanical orthotic element designed to constitute a contact surface with at least the patient's lower limbs and a neuromuscular stimulation element including at least a pair of electrodes designed to act on the muscle concerned of the patient's limb. The orthotic element comprises two orthoses including each three segments designed to co-operate with respectively the patient's thigh, leg and foot. The first segment is connected at one of its ends through a first motorized articulation to an element designed to co-operate with the patient's body at his hips and at its other end through a second motorized articulation to one of the ends of the second segment. The other end of the second segment is linked through a third motorised articulation to the third segment.

Document EP-A-475735 discloses an orthopaedic reeducation equipment comprising a reversible motorisation assembly attachable to a mobilising splint. The assembly comprises a housing fastened to the structure and mounted rotatably on the joint pin which is fixed to the support, a large toothed ring immobilised on the pin, an electric motor with two directions of rotation mounted in the housing, and a reduction mechanism mounted in the housing and interposed between the electric motor and the toothed ring which it engages.

Document EP-A-510840 discloses an orthosis for stretching tissue that limits compressive forces on the soil tissue around a joint during the movement of the joint. The orthosis may be operated by a manual plus electric drive cycling it between extremes of motion.

This invention proposes to provide an articulated joint for a walker, for example part of a knee brace type device, or also for application on ankles, arms or other parts of the body comprising joints, which is able to eliminate or at least reduce the disadvantages described above, being equipped with power-driving means.

In this case, the walker that allows the limb to be held in a condition of safety can also become a means of passive rehabilitation, thus avoiding the need to use additional equipment.

The invention also proposes to provide a power-driven articulated joint that is easy to produce in such a way as to be economically advantageous.

This is achieved by means of a power-driven joint with the features described in the main claim.

The dependent claims describe advantageous embodiments of the invention.

The power-driven joint according to the invention therefore comprises a pair of reciprocally hinged discs, each being integral with a respective upright fixed in turn to the user's limb.

In the case of the leg, each upright is fixed to the femoral sector and to the tibial sector, but advantageously the device also foresees the use of any other kind of joint and for any type of articular trauma, to the lower or upper limbs.

In the post-traumatic treatment of joints, it is normally indispensable for the angular extension between one disc and the other to be limited within a range that gradually increases as time passes; a different degree of control must also be ensured for angular extension movement with respect to flexure movement.

The adjustment of these oscillatory distances must be carried out by specialised personnel or, following precise instructions of the doctor, by the user who can intervene directly on his/her brace.

The articulated joint must therefore present devices which are easily adjustable and easy to maneuver.

The invention in question proposes to motorize this type of joint by using and electrical type motor, and more specifically the stepping type motor, which can generate controlled angular movements of one upright with respect to the other.

The motor fitted on the joint can be fixed by very easy to use means on the device, for example on one of the uprights, and foresees that its shaped operating pin is directly inserted in a corresponding shaped hole of the joint, thus angularly moving one upright with respect to the other.

The angular movements imparted can be managed and controlled by a PLC or by another control system on which it is possible to set both the size of the angles and the speed of the movement.

The main advantages of this solution, in addition to those deriving from the construction simplicity rather than the traditional complexity and the difficulty in using known kinetic type equipment, are a considerable saving in costs, since the same device that allows support of the limb in the post-operative period also makes it possible to carry out cycles of passive rehabilitation with controlled angular movements at an intensity that gradually increases as time passes.

### DESCRIPTION OF THE DRAWINGS

Other features and advantages of the invention will become evident on reading the following description of one embodiment of the invention, given as a non-binding example, with the help of the drawings shown in the attached pages, in which:

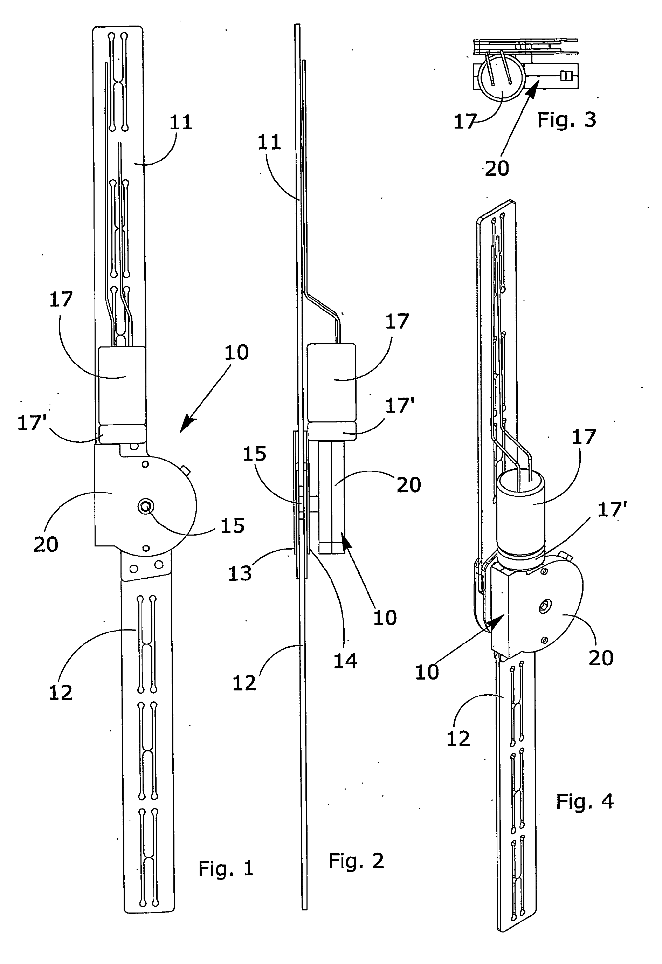

- figure 1 represents a side schematic view of an articulated joint fitted between the respective uprights and equipped with the power-driven system according to the invention;
- figure 2 represents a front schematic view;
- figure 3 is a plan schematic view of the power-driven joint;
- figure 4 is an axonometric view of a joint complete with uprights and equipped with power-driving means;

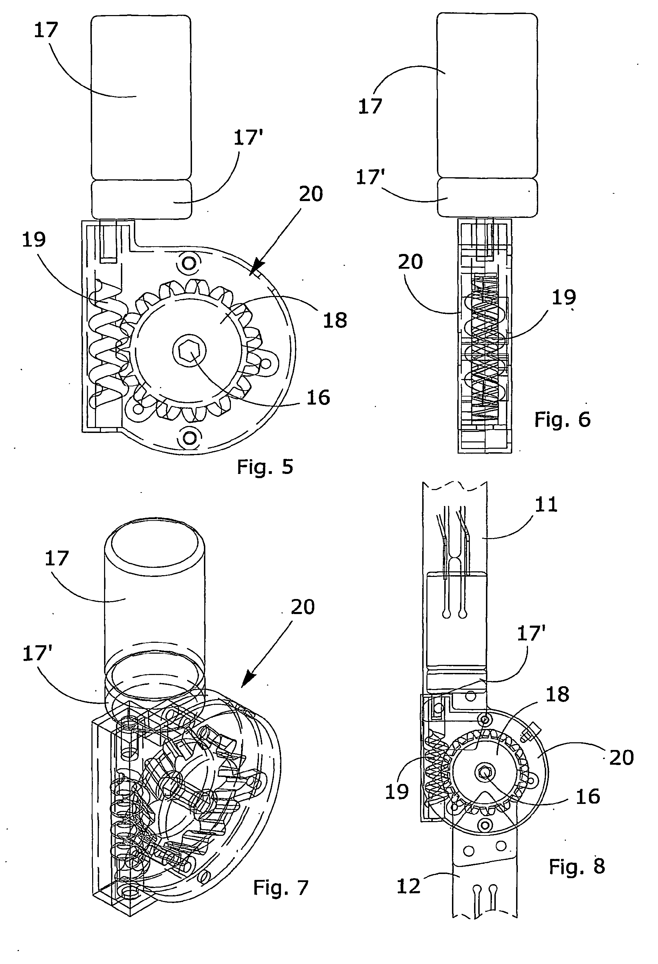

- figure 5 shows the power-driving means and its gears partially transparent;
- figure 6 shows a front schematic view;
- figure 7 shows an axonometric schematic view;
- figure 8 represents a detailed view of an articulated joint equipped with power-driving means;

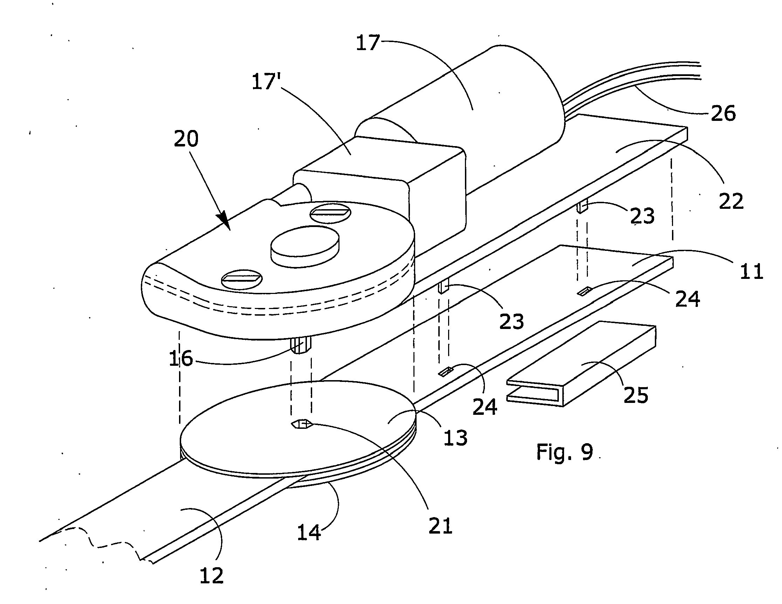

- figure 9 shows an axonometric exploded view of the joint fitted on the uprights and equipped with power-driving means.

### DESCRIPTION OF ONE EMBODIMENT OF THE INVENTION

The articulated joint 10 according to the invention is part of a post-operative knee brace or other equipment comprising uprights 11 and 12 which, in the case of the leg, are respectively fixed to the femoral sector and to the tibial sector, but which advantageously also foresees the use of articulated joints of any kind and for any type of articular trauma of the upper or lower limbs.

According to the invention, the articulated joint 10 comprises at least one pair of discs 13 and 14, which may be equipped with stop teeth, respectively integral with the femoral upright 11 and the tibial upright 12, considering that the terms used here with reference to the knee can also refer to other joints of the upper or lower limbs.

According to the invention, the joint presents means designed for the reciprocal angular movement of the two uprights for adjustment of the angle of flexure and the angle of extension.

The two discs 13 and 14 are connected and integral with the femoral upright 11 and the tibial uprights 12, and are connected together by means of a rotation pin 15 common to both.

The joint may also comprise means of elastic loading, consisting for example of a helical spring, one end of which is directly or indirectly in contact with the central pin of the joint and the other end in contact with the pin or part of the upright to which it is directly connected.

This means of elastic loading can advantageously be inserted in a specific housing in the body of the strip.

As stated above, the joint is normally equipped with teeth that, at the end of the allowed movement, strike an appropriate pin, thus limiting the angular movement of the tibial upright with respect to the femoral upright.

The independent movement of the pins inside other notches available in the respective circumferential slots in the joint ensures a different angular movement and thus an adjustment of the angle of flexure and the angle of extension.

According to the invention, the joint can be equipped with power-driving means which allow the angular movement of the uprights in order to move the body joint on which the device is fitted.

In particular, the power-driving means consist of a pinion 16 of a unit which is kinematically connected to an electrical type motor 17 and to a reduction gear unit 17'.

More specifically, the pinion 16 is part of and integral with a toothed pulley 18 which engages with a worm screw 19 driven by the shaft of the motor 17.

The worm screw 19 is positioned at a tangent to the pulley 18 and both the screw and the pulley are enclosed in a container 20 which has approximately the same surface extension as the discs 13 and 14 over which it is positioned.

The pinion 16, which presents a longitudinally shaped conformation, for example hexagonal, triangular, polygonal, toothed or similar, is inserted in an appropriate corresponding housing 21 in the pin 15 of the joint, and more specifically in a housing of the pin integral with one of the two uprights.

In the case in question, the motor 17 and the drive and reduction unit enclosed in the container 20 are fitted on the femoral upright 11.

The pinion 16 is in turn shaped so as to penetrate the corresponding female hole 21 of the pin 15, which is therefore integral with the tibial upright 12.

In this way a rotation of the pinion 16 causes an angular movement of the tibial upright with respect to the femoral upright.

The drive unit, consisting of the motor 17 and the container 20 holding the kinematic movement transmission organs, is fitted on a support plate 22 equipped on the opposite side with protruding centering teeth 23.

The teeth 23 are shaped and arranged so they can be inserted in corresponding housing 24 in the upright 11.

A locking clip 25 with a substantially "C" shaped cross-section, or another similar shape, clamps the plate 22 on the upright 11.

The motor 17 is connected by appropriate cabling 26 to a power takeoff which can be represented by a storage battery or a network source, and the motor can be connected to a PLC control unit or the like, which acts by keyboard commands.

In this way it is possible to control the angle of movement of the uprights, starting from narrow angles at the start of treatment with a gradually increasing intensity in relation to time.

It is also possible to control the speed of the movements according to requirements.

The joint and the parts that make up the movement system can be made from lightweight metal alloy or from high-resistance composite plastic.

According to the invention, the power-driving system may also be removable, being fitted and held in place only when the movement system is actually being used.

It is also possible to foresee that, with an appropriate modification to the hinge pin of the upright joint, all existing similar brace devices can be equipped with the power-driving system.

The invention is described above with reference to a preferred embodiment. It is nevertheless clear that the invention is susceptible to numerous variations that lie within the framework of technical equivalents.

## Claims

1. An articulated joint (10) for a post-operative knee brace or for articular walkers in general, of the type designed to form an articulated connection between a pair of uprights (11, 12) each of which can be applied to a respective sector of a body joint to be treated, comprising power-driving means (16, 17, 17') for the angular movement of one upright with respect to the other, said power driving means are fitted at a hinge point connecting the two uprights (11, 12), whereby said power-driving means comprise a shaped pinion (16) which is part of a unit kinematically connected to an electrical type motor (17) and to a reduction gear unit (17'), and whereby said motor (17) and a drive and reduction unit enclosed in a container (20) are fitted on one of the uprights (11), **characterised in that** said pinion (16) is in turn shaped so as to penetrate a corresponding female hole (21) of a rotation pin (15) of the joint, which is integral with the other upright (12).

2. An articulated joint according to claim 1 **characterised in that** said pinion (16) is part of and integral with a toothed pulley (18) which engages with a worm screw (19) driven by the shaft of the motor (17).

3. An articulated joint according to claim 2 **characterised in that** said worm screw (19) is positioned at a tangent with respect to the pulley (18) and both the screw and the pulley are enclosed in said container (20) which has approximately the same surface extension as a pair of joint discs (13, 14) over which it is positioned.

4. An articulated joint according to any of the foregoing claims **characterised in that** the pinion (16) presents a longitudinally shaped conformation, for example hexagonal, triangular, polygonal, toothed or similar.

5. An articulated joint according to any of the foregoing claims **characterised in that** the drive unit comprising said motor (17) and said container (20) holding the kinematic movement transmission organs, is fitted on a support plate (22) equipped on the opposite side with protruding centering teeth (23).

6. An articulated joint according to claim 5, **characterised in that** said teeth (23) are shaped and arranged so they can be inserted in corresponding housings (24) in the upright (11).

7. An articulated joint according to claim 5 or 6, **characterised in that** a locking clip (25) with a substantially "C" shaped cross-section, or another similar shape, clamps the plate (22) on the respective upright (11).

8. An articulated joint according to any of the foregoing claims **characterised in that** said motor (17) is connected by appropriate cabling (26) to a power takeoff which can be represented by a storage battery or a network source, and if necessary to a PLC control unit or the like, which acts by keyboard commands.

## Patentansprüche

1. Artikuliertes Gelenk (10) für eine postoperative Kniestütze oder für artikulierte Geher ganz allgemein, vom Typ, welches eine artikulierte Verbindung zwischen einem Paar von Stützen (11, 12) ausbildet, von denen jede an einem jeweiligen Abschnitt eines Körpergelenks eingesetzt werden kann, welches zu behandeln ist, mit einem Antriebsmittel (16, 17, 17') für die winkelmäßige Bewegung einer Stütze relativ zu der anderen Stütze, wobei die Antriebsmittel an einem Gelenkpunkt eingesetzt sind, der die beiden Stützen (11, 12) miteinander verbindet, wobei die Antriebsmittel einen geformten Abtrieb (16) umfassen, der Teil einer Einheit ist, die kinematisch mit einem Motor (17) vom elektrischen Typ und mit einer Reduktionsgetriebeeinheit (17') verbunden ist, und wobei der Motor (17) und die Antriebs- und Reduktionseinheit, die von einem Behälter (20) umschlossen sind, an einer der Stützen (11) angebracht sind, **dadurch gekennzeichnet, dass** der Abtrieb (16) derart ausgeformt ist, dass er eine korrespondierende Kontaktöffnung (21) eines Rotationsstiftes (15) des Gelenks durchdringt, welches mit der anderen Stütze (12) integral ausgebildet ist.

2. Artikuliertes Gelenk nach Anspruch 1, **dadurch gekennzeichnet, dass** der Abtrieb (16) ein Teil einer und integriert mit einer gezahnten Scheibe (18) ist, die mit einem Schneckentrieb (19) in Eingriff steht, welcher durch die Achse des Motors (17) angetrieben ist.

3. Artikuliertes Gelenk nach Anspruch 2, **dadurch gekennzeichnet, dass** der Schneckentrieb (19) mit Bezug zu der Scheibe (18) an einer Tangente positioniert ist und dass beide, der Schneckentrieb und die Scheibe von dem Behälter (20) umschlossen sind, der annähernd die gleiche Oberflächenerstreckung aufweist wie ein Paar von Gelenkscheiben (13, 14), oberhalb deren er positioniert ist.

4. Artikuliertes Gelenk nach irgendeinem der vorherigen Ansprüche, **dadurch gekennzeichnet, dass** der Abtrieb (16) eine längsgerichtete geformte Gestalt aufweist, zum Beispiel hexagonal, dreieckig, polygonal, gezahnt oder ähnliches.

5. Artikuliertes Gelenk nach irgendeinem der vorherigen Ansprüche, **dadurch gekennzeichnet, dass** die Antriebseinheit, die den Motor (17) und den Behälter (20) umfasst, der die kinematischen Bewegungsübertragungsorgane beinhaltet, auf eine Tragplatte (22) gesetzt ist, die auf der gegenüberliegenden Seite mit überstehenden Zentrierungszähnen 823) versehen ist.

6. Artikuliertes Gelenk nach Anspruch 5, **dadurch gekennzeichnet, dass** die Zähne (23) derart ausgeformt und angeordnet sind, dass diese in korrespondierende Aufnahmen (24) in der Stütze (11) einsetzbar sind.

7. Artikuliertes Gelenk nach Anspruch 5 oder 6, **dadurch gekennzeichnet, dass** eine Verschlußklammer (25) mit einem im Wesentlichen "C-förmigen" Querschnitt oder mit einer ähnlichen Form die Platte (22) an der jeweiligen Stütze (11) festklammert.

8. Artikuliertes Gelenk nach irgendeinem der vorherigen Ansprüche, **dadurch gekennzeichnet, dass** der Motor (17) mit einem geeigneten Kabel (26) mit einer Energiequelle verbunden ist, die durch eine Speicherbatterie oder eine Netzquelle ausgebildet ist, und falls erforderlich mit einer programmierbaren Logiksteuerungseinheit oder ähnlichem, die auf Befehlseingabe über eine Tastatur funktioniert.

## Revendications

1. Articulation (10) pour orthèse de genou post-opératoire ou d'une façon générale pour déambulateurs articulés, du type conçu pour former une connexion articulée entre une paire de montants (11, 12) qui peuvent chacune être appliquée à une partie respective d'une articulation du corps à traiter, ladite articulation comprenant des moyens d'entraînement motorisés (16, 17, 17') pour le mouvement angulaire d'un montant par rapport à l'autre, lesdits moyens d'entraînement motorisés sont montés au niveau d'un point charnière entre les deux montants (11, 12), lesdits moyens d'entraînement motorisés comprenant un pignon conformé (16) qui fait partie d'une unité cinématiquement raccordée à un moteur de type électrique (17) et à une unité formant réducteur (17'), et ledit moteur (17) et une unité d'entraînement et de réduction enfermée dans un boîtier (20) étant montés sur l'un des montants (11), **caractérisée en ce que** ledit pignon (16) est conformé pour pénétrer dans un trou femelle correspondant (21) d'un pivot de rotation (15) de l'articulation, lequel est solidaire de l'autre montant (12).

2. Articulation selon la revendication 1, **caractérisée en ce que** ledit pignon (16) fait partie d'une poulie dentée (18) dont il est solidaire et qui s'engage avec une vis sans fin (19) entraînée par l'arbre du moteur (17).

3. Articulation selon la revendication 2, **caractérisée en ce que** ladite vis sans fin (19) est positionnée de façon à être tangente à la poulie (18) et la vis et la poulie sont toutes les deux enfermées dans ledit boîtier (20) qui s'étend approximativement sur la même surface qu'une paire de disques d'articulation (13, 14) sur lesquels il est positionné.

4. Articulation selon l'une quelconque des revendications précédentes, **caractérisée en ce que** le pignon (16) présente une conformation longitudinale, par exemple hexagonale, triangulaire, polygonale, dentée ou similaire.

5. Articulation selon l'une quelconque des revendications précédentes, **caractérisée en ce que** l'unité d'entraînement, comprenant ledit moteur (17) et ledit boîtier (20) qui contient les organes de transmission de mouvement cinématique, est montée sur une plaque de support (22) équipée du côté opposé de dents de centrage saillantes (23).

6. Articulation selon la revendication 5, **caractérisée en ce que** lesdites dents (23) sont conformées et disposées de façon à pouvoir être insérées dans des logements correspondants (24) ménagés dont le montant (11).

7. Articulation selon la revendication 5 ou 6, **caractérisée en ce qu'**une prince de blocage (25) de section sensiblement en forme de « C », ou d'une autre forme similaire, serre la plaque (22) sur le montant respectif (11).

8. Articulation selon l'une quelconque des revendications précédentes, **caractérisée en ce que** ledit moteur (17) est raccordé par un câblage approprié (26) à une alimentation qui peut être représentée par une batterie ou une source du réseau et, si nécessaire, à une unité de commande PLC ou analogue, qui agit par des commandes au clavier.
